# EUROPÄISCHE PATENTSCHRIFT

(11) **EP 4 429 607 B1**
(45) Veröffentlichungstag und Bekanntmachung des Hinweises auf die Patenterteilung: **06.08.2025**
(21) Anmeldenummer: 22830418.4
(22) Anmeldetag: 02.12.2022
(51) Int. Cl.: A61F 5/01

(54) **ORTHESENTEIL FÜR EINE ORTHESE, BAUSATZ ZUR HERSTELLUNG DES ORTHESENTEILS UND ORTHESE**
ORTHOSIS PART FOR AN ORTHOSIS, KIT FOR THE MANUFACTURE OF THE ORTHOSIS PART AND ORTHOSIS
ÉLÉMENT D'ORTHÈSE POUR UNE ORTHÈSE, KIT POUR LA FABRICATION DE L'ÉLÉMENT D'ORTHÈSE ET DE L'ORTHÈSE

(30) Priorität: 02.12.2021 DE 202021106600 U
(43) Veröffentlichungstag der Anmeldung: 18.09.2024
(73) Patentinhaber: HKK Bionics GmbH, 89075 Ulm (DE)
(72) Erfinder: HEPP, Dominik, 89343 Jettingen-Scheppach (DE)
(74) Vertreter: Hentrich Patent- & Rechtsanwaltspartnerschaft mbB
(86) Internationale Anmeldenummer: PCT/EP2022/084216
(87) Internationale Veröffentlichungsnummer: WO 2023/099737

(56) Entgegenhaltungen:
- EP-A1- 1 265 567
- WO-A1-2011/137999
- DE-U1- 202015 008 342
- US-A- 2 545 452
- US-A- 4 144 881
- US-A1- 2014 031 732
- US-A1- 2021 290 420
- US-B2- 10 864 100

## Beschreibung

Die Erfindung betrifft ein Orthesenteil für eine Orthese, umfassend ein erstes Orthesenglied, ein zweites Orthesenglied, ein Kopplungsteil, durch welches das erste Orthesenglied mit dem zweiten Orthesenglied begrenzt beweglich verbunden ist, und ein Rückstellelement, welches einenends mit dem ersten Orthesenglied und anderenends mit dem zweiten Orthesenglied verbunden ist, welches aus einer Legierung umfassend Titan und Nickel gebildet ist, und welches aufgrund seiner elastischen Materialeigenschaften dann eine Rückstellkraft bereitstellt, wenn das erste Orthesenglied relativ bezüglich dem zweiten Orthesenglied ausgelenkt wird.

Die Erfindung betrifft außerdem einen Bausatz zur Herstellung eines solchen Orthesenteils und eine Orthese mit einem solchen Orthesenteil.

Eine Orthese mit Orthesengliedern ist beispielsweise aus den Druckschriften EP 3 263 086 A1, EP 3 459 505 A1 und EP 3 711 718 A1 der Anmelderin bekannt. Die darin gezeigten Orthesen sind Handorthesen, welche mit einem Antrieb ausgestattet sind, um die Orthese aktiv zu betätigen.

Die US 2014 / 0 142 482 A1 beschreibt eine Knieorthese, bei der die beiden Orthesenglieder mittels eines Stapels aus Blattfedern miteinander verbunden sind, wobei der Stapel aus Blattfedern aus NiTi selbst oder einer NiTi-Legierung gebildet sind. Die Blattfedern dienen als Rückstellelement, wobei kein zusätzliches Kopplungsteil vorhanden ist, um die beiden vom Blattfederstapel verbundenen Orthesenglieder zwischen zwei Begrenzungen relativ zueinander zu bewegen.

In der US 2015 / 0 290 015 A1 wird eine Fußgelenkorthese gezeigt, die aus einer Formgedächtnislegierung (SMA für "shape memory alloy") gebildet ist, die aber aufgrund ihrer Formgebung nur einseitig wirkt. Das Rückstellelement verbindet die beiden Orthesenglieder miteinander. Es fehlt aber eine definierte Begrenzung für die Relativ-Bewegung der beiden Orthesenglieder zueinander.

In der US 2019 / 0 133 803 A1 wird eine Orthese zur Korrektur eines hallux valgus beschrieben. Um eine Rückstellkraft auf den Hallux auszuüben, wird eine superelastische Legierung genutzt. Auch hier wird neben dem Rückstellelement kein weiteres Element für eine Begrenzung der Bewegbarkeit der einzelnen Orthesenglieder bereitgestellt.

In der US 4,144,881 A wird ein Orthesenteil gezeigt, das sich aus einer Mehrzahl von Einzelgliedern zusammensetzt, die eine T-förmige Zunge besitzen, welche in eine T-förmige Nut eingeschoben werden können. In der Variante nach Figur 6 der Druckschrift kann diese Konfiguration auch ergänzt werden um ein Rückstellelement, nämlich um eine Feder, die aus einem Federstahl gebildet ist. Ausgehend davon ist es Aufgabe der vorliegenden Erfindung, ein Orthesenteil, einen Bausatz für ein Orthesenteil und eine Orthese bereitzustellen, die eine verbesserte Unterstützung für die Bewegung, insbesondere auch für Begrenzung der Bewegung bereitstellen.

Die US 2 545 452 A beschreibt einen künstlichen Finger nach Art einer Prothese. Die Druckschriften WO 2011 / 137 999 A1, US 10 864 100 B2, US 4 144 881 A, US 2014 / 0 031 732 A1, US 2021 / 0 290 420 A1 werden Orthesen und Orthesenteile beschrieben. Die WO 2011 / 137 999 A1 beschreibt ein Orthesenteil gemäß dem Oberbegriff des Anspruchs 1.

Diese Aufgabe wird mit einem Orthesenteil mit den Merkmalen des Anspruchs 1, durch einen Bausatz mit den Merkmalen des Anspruchs 14 und durch eine Orthese mit den Merkmalen des Anspruchs 15 gelöst. Vorteilhafte Ausgestaltungen mit zweckmäßigen Weiterbildungen der Erfindung sind in den abhängigen Ansprüchen angegeben.

Das eingangs genannte Orthesenteil zeichnet sich durch das Rückstellelement aus, das im Falle einer aktiven, d. h. mit einem Antrieb versehenen, Orthese die Bewegung unterstützt. Bei einer passiven Orthese wird die vom Orthesennutzer aufgebrachte Kraft unterstützt oder der von vom Benutzer aufgebrachten Kraft wird entgegengewirkt.

Aufgrund der gewählten Legierung umfassend Titan und Nickel, insbesondere wenn letztere zu gleichen Anteilen binär in der Legierung vorliegen (Nitinol), können die superelastischen Eigenschaften für die Rückstellkraft genutzt werden.

Es hat sich zudem als besonders vorteilhaft erwiesen, wenn die Legierung außerdem Kobalt umfasst, wobei insbesondere die Legierung von 1 bis 5 Gewichts- % Kobalt umfasst, wobei Titan und Nickel vorzugsweise zu gleichen Teilen vorliegen. Um eine noch bessere Steifigkeit hervorzurufen, ist es bevorzugt, wenn der Kobaltanteil 1 bis 2 Gewichts-% der Legierung ausmacht.

Um die superelastischen Eigenschaften - wohl gemerkt nicht die thermisch induzierte Gedächtnisfunktion - auszunutzen, hat es sich als vorteilhaft erwiesen, wenn die Legierung des Rückstellelements gewonnen ist aus der austenitischen Phase und somit als Austenit oder überwiegend als Austenit vorliegt.

Zur sicheren Positionierung des Rückstellelements ist erfindungsgemäß vorgesehen, dass in dem ersten Orthesenglied eine erste Tasche ausgebildet ist, wenn in dem zweiten Orthesenglied eine zweite Tasche ausgebildet ist, und wenn das Rückstellelement einenends in der ersten Tasche und anderenends in der zweiten Tasche aufgenommen ist.

Die Elastizität lässt sich gezielt einstellen, erfindungsgemäß dann, wenn in jeder der Taschen ein Teil eines zweiten Rückstellelements aufgenommen ist, und wenn das zweite Rückstellelement aus derselben Legierung wie das Rückstellelement besteht. Aufgrund der identischen Legierung kann die wirkende Rückstellkraft sehr gezielt eingestellt werden. Es ist die Möglichkeit vorhanden, dass auch mehr als zwei Rückstellelemente in den Taschen platziert werden, sodass auch drei oder mehr Rückstellelemente Einsatz finden können, um die Steifigkeit und damit die Elastizität gezielt einzustellen.

Das Rückstellelement und das zweite Rückstellelement oder auch die weiteren Rückstellelemente können dabei dieselbe Querschnittsform aufweisen. Zur Einstellung der Rückstellkraft kann also dieselbe Form mehrfach Verwendung finden.

Zur gezielten Einstellung der Rückstellkraft kann beispielsweise die Querschnittsfläche des Rückstellelements verändert werden, sodass beispielsweise ein dünnes Blech durch ein dickes Blech oder ein kleiner Durchmesser beim Rückstellelements durch einen größeren Durchmesser beim Rückstellelement ersetzt werden kann, und umgekehrt.

Es ist auch die vorteilhafte Möglichkeit gegeben, dass sich die Querschnittsform des Rückstellelements von der Querschnittsform des zweiten Rückstellelements unterscheidet. Hierbei kann gezielt eine Kombination unterschiedlicher Formen genutzt werden, um die gewünschte Rückstellkraft einzustellen. Als Formen kommen beispielsweise in Betracht: Band, Draht, Rohr, ovale Formen, entweder als gerades / gestrecktes Material oder als gekrümmtes / gebogenes Material. Unterschiedliche Formen können dann kombiniert werden, wobei zum Beispiel zweimal die Form eines Drahtes und einmal die Form eines Bandes Einsatz finden könnte.

Es hat sich als vorteilhaft herausgestellt, wenn die Querschnittsform des Einen aus Rückstellelement und zweitem Rückstellelement im Wesentlichen rechteckig ist, und wenn die Querschnittsform des Anderen aus Rückstellelement und zweitem Rückstellelement im Wesentlichen elliptisch, insbesondere kreisförmig ist.

Zur Gewichtsreduzierung ist die Möglichkeit vorhanden, dass das mindestens eine Rückstellelement im Wesentlichen röhrenförmig gebildet ist.

Vorzugsweise liegt das Rückstellelement separat vom Kopplungsteil vor, damit das Rückstellelement als austauschbares und/oder ergänzbares Bauteil realisiert werden kann. So lässt sich die wirkende Rückstellkraft individuell einstellen.

Die sichere Lagerung des Rückstellelements lässt sich dadurch bewirken, dass in dem Kopplungsteil ein Tunnel für das Rückstellelement ausgebildet ist, und dass das mindestens eine Rückstellelement durch den Tunnel hindurchgeführt ist.

Es ist von Vorteil, wenn in dem Kopplungsteil ein Durchtritt für ein weiteres Rückstellelement ausgebildet ist, und wenn das weitere Rückstellelement durch den Durchtritt hindurchgeführt ist. Auf diese Weise liegen also zwei definierte Positionen, nämlich einerseits durch den Tunnel und andererseits durch den Durchtritt, für die Anordnung der Rückstellelemente vor, womit sich die Rückstellkraft gezielt einstellen oder auch verändern lässt.

Zur Einstellung der Rückstellkraft ist es von Vorteil, wenn auch das weitere Rückstellelement aus derselben Legierung wie das Rückstellelement besteht.

Es ist auch die Möglichkeit vorhanden, dass der Durchtritt in dem Kopplungsteil für die Durchführung eines Krafteinleitungsmittels zur aktiven Betätigung der Orthese ausgebildet ist, wobei dann das Krafteinleitungsmittel durch den Durchtritt hindurchgeführt ist. Als Krafteinleitungsmittel kommt beispielsweise ein Nitinol-Draht oder ein Draht aus NiTiCo in Betracht.

Um die Orthese an die unterschiedlichen Gegebenheiten des menschlichen Körpers anpassen zu können, hat es sich als vorteilhaft erwiesen, wenn das Kopplungsteil aus einer Mehrzahl von Koppelgliedern gebildet ist, die untereinander begrenzt beweglich miteinander verbunden sind.

Um über das Kopplungsteil hinweg die Rückstellkraft bereitstellen zu können, hat es sich als vorteilhaft erwiesen, wenn sich der Tunnel für das Rückstellelement durch alle Koppelglieder des Kopplungsteils hindurch erstreckt.

Zusätzlich ist die Möglichkeit gegeben, dass sich auch ein Durchtritt für ein weiteres Rückstellelement und/oder für ein Krafteinleitungsmittel zur aktiven Betätigung der Orthese durch alle Koppelglieder des Kopplungsteils hindurch erstreckt.

Eine zuverlässige, begrenzt bewegliche Verbindung lässt sich beispielsweise mit einem Bajonettverschluss bilden. In diesem Zuge ist es daher von Vorteil, wenn jedes der Koppelglieder eine an einer ersten Stirnseite ausgebildete Bajonettkulisse aufweist, wenn jedes der Koppelglieder einen zu der Bajonettkulisse korrespondierenden Bajonetthaken aufweist, der an einer der ersten Stirnseite gegenüberliegenden zweiten Stirnseite ausgebildet ist, und wenn zwischen je zwei der Koppelglieder oder zwischen einem der Koppelglieder und einem der

Orthesenglieder eine begrenzt bewegliche Bajonettverbindung ausgebildet ist.

Es ist die Möglichkeit gegeben, dass das Koppelteil einen Verschluss ausbildet, wobei ein erster Verschlussteil des Verschlusses einteilig mit dem ersten Orthesenglied gebildet ist, und wobei ein zum ersten Verschlussteil passender, zweiter Verschlussteil des Verschlusses einteilig mit dem zweiten Orthesenglied gebildet ist. Damit lässt sich das Orthesenteil mit einer geringeren Anzahl an Bauteilen bilden, wobei der Vorteil erhalten bleibt, dass das Rückstellelement noch immer als separates Bauteil vorliegt und zur Einstellung oder Veränderung der Rückstellstraft austauschbar und/oder ergänzbar ist. Der Verschluss ist beispielsweise durch eine Bajonettverbindung realisiert, bei der ein Bajonetthaken dem Einen der beiden Orthesenglieder zugeordnet ist, und bei der eine zum Bajonetthaken korrespondierende Bajonettkulisse dem Anderen der beiden Orthesenglieder zugeordnet ist.

Der erfindungsgemäße Bausatz zur Herstellung eines Orthesenteils umfasst mindestens ein erstes Orthesenglied, mindestens ein zweites Orthesenglied, und gegebenenfalls mindestens ein Kopplungsteil, wobei das erste Orthesenglied mit mit dem zweiten Orthesenglied verbindbar ist. Der Bausatz umfasst ferner mindestens ein Rückstellelement, welches aus einer Legierung umfassend Titan und Nickel gebildet ist, und welches aufgrund seiner elastischen Materialeigenschaften dann eine Rückstellkraft bereitstellt, wenn im montierten Zustand des Orthesenteils das erste Orthesenglied relativ bezüglich dem zweiten Orthesenglied ausgelenkt wird.

Es ist auch beim Bausatz die Möglichkeit gegeben, dass das Koppelteil einen, insbesondere zwischen zwei Anschlägen bewegbaren, Verschluss ausbildet, wobei ein erster Verschlussteil des Verschlusses einteilig mit dem ersten Orthesenglied gebildet ist, und wobei ein zum ersten Verschlussteil passender, zweiter Verschlussteil des Verschlusses einteilig mit dem zweiten Orthesenglied gebildet ist. Damit lässt sich der Bausatz mit einer geringeren Anzahl an Bauteilen bilden, wobei der Vorteil erhalten bleibt, dass das Rückstellelement noch immer als separates Bauteil vorliegt und zur Einstellung oder Veränderung der Rückstellstraft austauschbar und/oder ergänzbar ist. Der Verschluss ist beispielsweise durch eine Bajonettverbindung realisiert, bei der ein Bajonetthaken dem Einen der beiden Orthesenglieder zugeordnet ist, und bei der eine zum Bajonetthaken korrespondierende Bajonettkulisse dem Anderen der beiden Orthesenglieder zugeordnet ist.

Auch hier ist die Legierung vorzugsweise aus Nitinol geformt. Die Legierung kann aber auch NiTiCo sein.

Die Verbindung mit dem Orthesenteil erläuterten Vorteile und vorteilhaften Ausgestaltungen und Wirkungen gelten in gleicher Weise für die erfindungsgemäße Orthese. Diese ist insbesondere für ein Gelenk einer der Extremitäten des menschlichen Körpers eingerichtet. Sie kann beispielsweise im Bereich der unteren Extremitäten genutzt werden. Es ist jedoch auch möglich, eine Orthese für eine der oberen Extremitäten mit einem solchen Orthesenteil zu bilden.

In diesem Zusammenhang ist daher die Möglichkeit gegeben, dass die Orthese als eine Handorthese gebildet ist, bei der das Orthesenteil einen Teil eines Fingerglieds bildet.

Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen als von der Erfindung umfasst und offenbart anzusehen, die in den Figuren nicht explizit gezeigt oder erläutert sind, jedoch durch separierte Merkmalskombinationen aus den erläuterten Ausführungen hervorgehen und erzeugbar sind.

Weitere Vorteile, Merkmale und Einzelheiten der Erfindung ergeben sich aus den Ansprüchen, der nachfolgenden Beschreibung bevorzugter Ausführungsformen sowie anhand der Zeichnungen. Dabei zeigen:
- Figur 1: eine schematische Darstellung eines Orthesenteils,
- Figur 2: eine schematische Darstellung einer Orthese, die in Form einer Handorthese vorliegt,
- Figur 3: ein Orthesenteil der Handorthese aus Figur 2 in einer Seitenansicht,
- Figur 4: das Orthesenteil aus Figur 3 in einer Schnittansicht,
- Figur 5: eine perspektivische Ansicht auf die erste Stirnseite eines Kopplungsteils,
- Figur 6: eine perspektivische Ansicht auf die zweite Stirnseite des Kopplungsteils aus Figur 5,
- Figur 7: eine der Figur 4 entsprechende Darstellung eines Orthesenteils mit einem weiteren Rückstellelement oder einem Krafteinleitungsmittel, das durch die Durchtritte hindurchgeführt ist, und
- Figur 8: eine der Figur 4 entsprechende Darstellung eines Orthesenteils, bei der das Rückstellelement durch die Durchtritte hindurchgeführt ist.

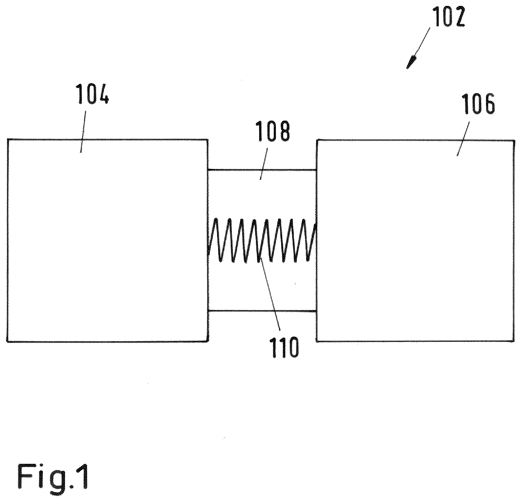

In Figur 1 ist schematisch ein Orthesenteil 102 für eine Orthese 100 gezeigt, welches aus einem ersten Orthesenglied 104 und einem zweiten Orthesenglied 106 besteht. Ferner ist ein Kopplungsteil 108 vorhanden, durch welches das erste Orthesenglied 104 mit dem zweiten Orthesenglied 106 begrenzt beweglich verbunden ist. Zusätzlich ist ein, insbesondere separat zum Kopplungsteil 108 vorliegendes, Rückstellelement 110 gezeigt, welches einenends mit dem ersten Orthesenglied 104 und anderenends mit dem zweiten Orthesenglied 106 verbunden ist. Dieses Rückstellelement 110 ist aus einer Legierung umfassend Titan und Nickel gebildet, die in dieser Legierung beispielsweise binär und zu gleichen Teilen vorliegen. Das Rückstellelement 110 stellt dabei aufgrund seiner elastischen Materialeigenschaften dann eine Rückstellkraft bereit, wenn das erste Orthesenglied 104 relativ bezüglich dem zweiten Orthesenglied 106 ausgelenkt wird. In diesem Zuge wird die Superelastizität der Legierung, insbesondere des Nitinols, genutzt.

Es ist jedoch die vorteilhafte Möglichkeit vorhanden, dass die Legierung zusätzlich Kobalt umfasst, welches in einem Anteil von ein bis fünf Gewichts-%, vorzugsweise mit einem Anteil von 1 bis 2 Gewichts-% in der Legierung vorliegt. Titan und Nickel liegen dabei jeweils zu gleichen Teilen vor. Um die Superelastizität nutzen zu können, liegt die Legierung des Rückstellelements als Austenit oder überwiegend als Austenit vor.

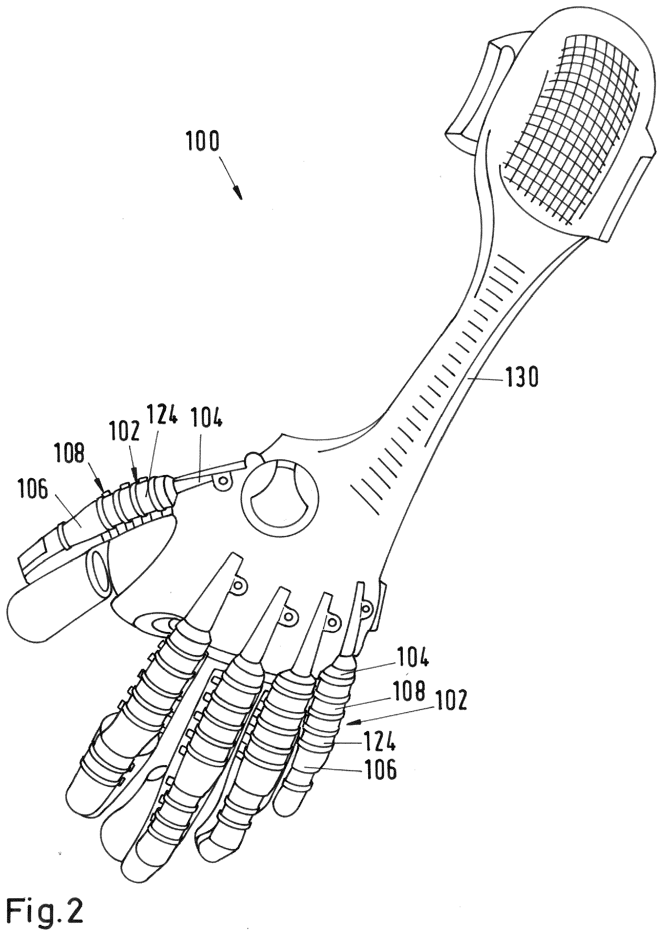

Das in Figur 1 schematisch dargestellte Orthesenteil 102 kann beispielweise in einer Orthese 100 nach Figur 2 Einsatz finden. Diese Orthese 100 ist eine Handorthese, wobei die vorliegende Erfindung nicht auf eine Orthese 100 für die oberen Extremitäten beschränkt ist. Das Orthesenteil 102 kann auch in einer Orthese 100 der unteren Extremitäten genutzt werden. Die Handorthese nach Figur 2 umfasst eine Unterarmschiene 130, die mit einem oder mehreren Bändern am Unterarm des Benutzers befestigt werden kann. Am distalen Teil der Unterarmschiene 130 sind eine Mehrzahl von Fingergliedern angeordnet, die dabei die Orthesenteile 102 bereitstellen. Hier ist zu erkennen, dass das Kopplungsteil 108, welches das erste Orthesenglied 104 mit dem zweiten Orthesenglied 106 verbindet, aus einer Mehrzahl von Koppelgliedern 124 gebildet ist, die vorliegend untereinander begrenzt beweglich miteinander verbunden sind. Das Kopplungsteil 108 kann jedoch auch integraler Bestandteil eines der beiden Orthesenglieder 104, 106 sein, sodass das Orthesenteil 102 insgesamt weniger Bauteile aufweist.

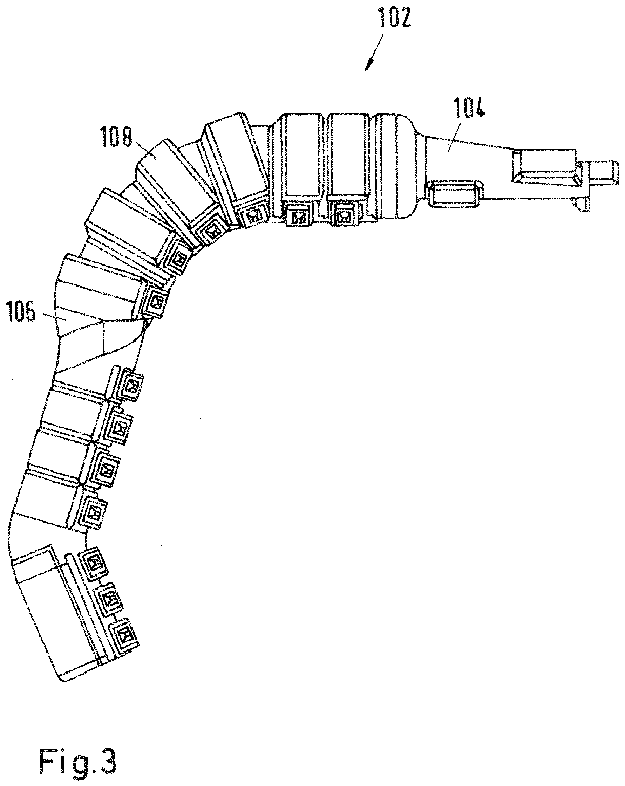

Eine Seitenansicht auf ein Orthesenteil 102 der Handorthese 100 aus Figur 2 ist in Figur 3 zu erkennen. Hierbei ist das erste Orthesenglied 104 bezüglich dem zweiten Orthesenglied 106 ausgelenkt und eine Rückstellkraft ist wirksam. Vorliegend liegt also mit anderen Worten eine Flexion des Fingerglieds vor, wobei die wirkende Rückstellkraft das Fingerglied in eine Extension (zurück-)drängt. Diese vorliegend gewählte Darstellung ist allerdings nur beispielhafter Natur. Es ist nämlich auch der umgekehrte Fall möglich, in welchem die Rückstellkraft dann wirksam ist, wenn das Fingerglied in die Extension gebracht wird, wobei die wirkende Rückstellkraft das Fingerglied dann in die Flexion (zurück-)drängt.

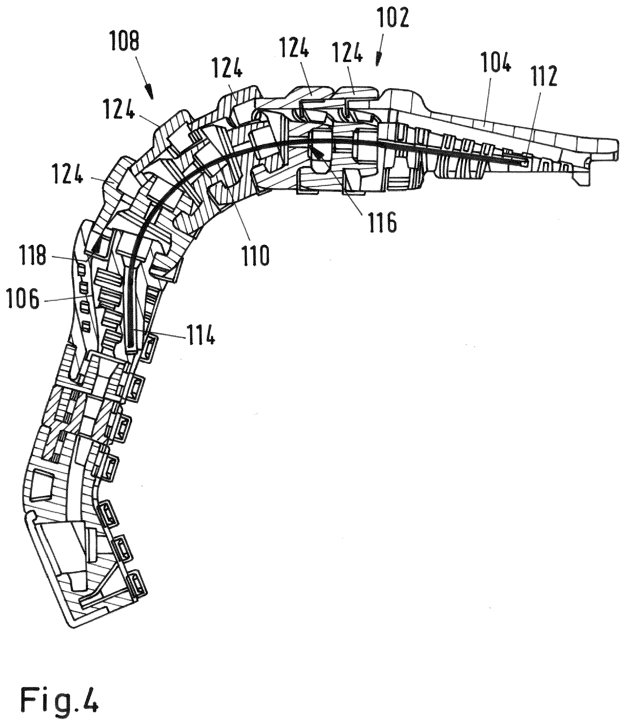

Eine Schnittansicht durch das Orthesenteil 102 nach Figur 3 ist beispielsweise in Figur 4 gezeigt, wobei zu erkennen ist, dass sich das Rückstellelement 110 zwischen dem ersten Orthesenglied 104 und dem zweiten Orthesenglied 106 erstreckt. Hierfür ist in dem ersten Orthesenglied 106 eine erste Tasche 112 ausgebildet, wobei in dem zweiten Orthesenglied 106 eine zweite Tasche 114 ausgebildet ist. Das Rückstellelement 110 ist einenends in der ersten Tasche 112 und anderenends in der zweiten Tasche 114 aufgenommen.

Das Rückstellelement 110 kann beispielsweise in Form eines Bandes, eines Drahtes, eines Rohres, als ovale Form, jeweils mit geradem / gestrecktem Material oder als gekrümmtes / gebogenes Material vorliegen. Über den Querschnitt des Materials lässt sich die Rückstellkraft gezielt einstellen.

Um die Rückstellkraft zwischen den beiden Orthesengliedern 102, 104 bereitstellen zu können, ist auch in dem Kopplungsteil 108 ein Tunnel 116 für das Rückstellelement 110 ausgebildet, wobei das Rückstellelement 110 durch den Tunnel 116 hindurchgeführt ist. Vorliegend weist jedes der Koppelglieder 124 des Koppelteils 108 einen solchen Tunnel 116 auf. Aus der Querschnittansicht nach Figur 4 wird zudem ersichtlich, dass auch ein Durchtritt 118 vorhanden ist, der sich durch das Kopplungsteil 108 und insbesondere auch durch die Mehrzahl der Koppelglieder 124 hindurch erstreckt.

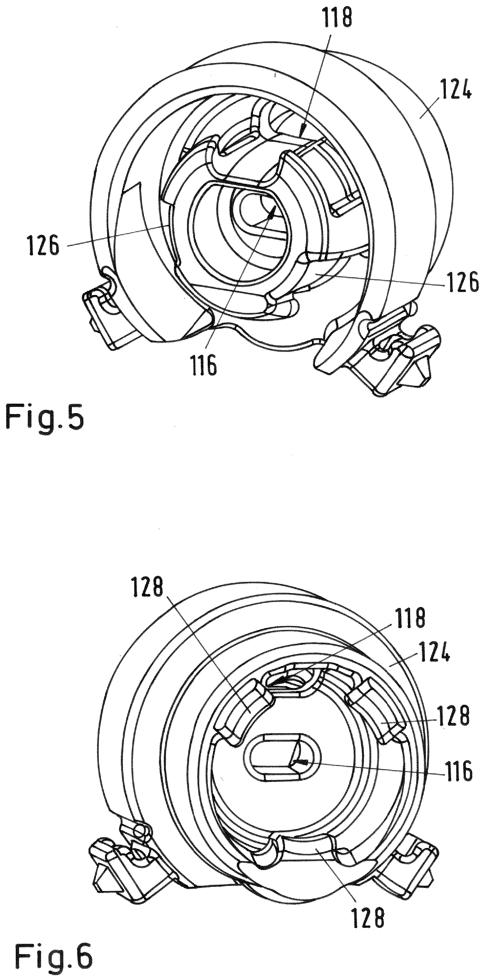

In den Figuren 5 und 6 ist eines der Koppelglieder 124 in einer Perspektive gezeigt, wobei festzustellen ist, dass jedes der Koppelglieder 124 eine an einer ersten Stirnseite ausgebildete Bajonettkulisse 126 aufweist, dass ferner jedes der Koppelglieder 124 einen zu der Bajonettkulisse 126 korrespondierenden Bajonetthaken 128 aufweist, der an einer der ersten Stirnseite gegenüberliegenden zweiten Stirnseite ausgebildet ist, und dass zwischen je zwei der Koppelglieder 124 oder zwischen einem der Koppelglieder 124 und einem der Orthesenglieder 104, 106 eine begrenzt bewegliche Bajonettverbindung ausgebildet ist. Diese begrenzte Beweglichkeit kann beispielsweise eine Rotation der beiden Koppelglieder 124 relativ zueinander bereitstellen. Es ist jedoch auch möglich, dass diese Bajonettverbindung eine begrenzt bewegliche Translation der beiden Koppelglieder 124 relativ bezüglich zueinander ermöglicht.

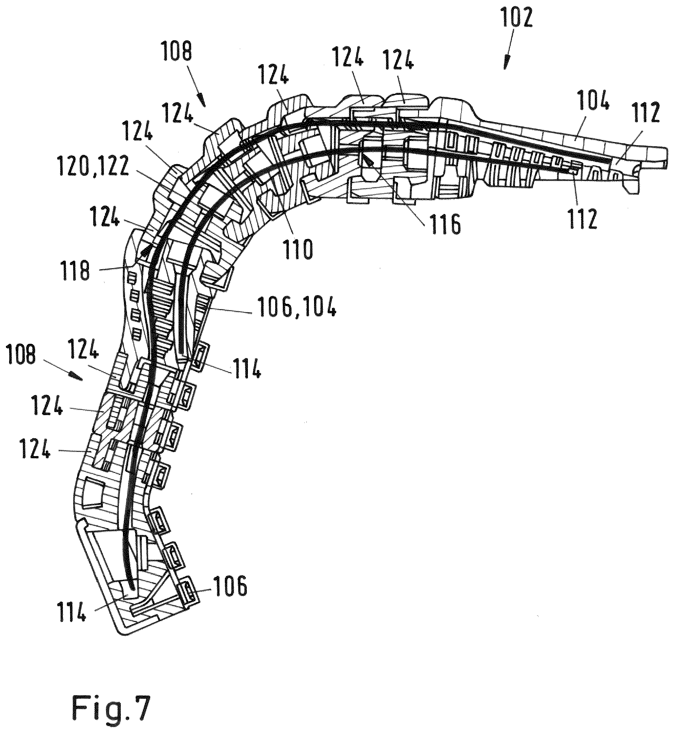

**In** Figur 7 wird der vorstehend bereits erwähnte Durchtritt 118 genutzt, um eine zusätzliche Rückstellkraft durch ein weiteres Rückstellelement 120 auf das Fingerglied der Orthese 100 ausüben zu können. Das weitere Rückstellelement 120 ist durch den Durchtritt 118 hindurchgeführt. Dabei umfasst das weitere Rückstellelement 120 ein Material, welches aus derselben Legierung wie das Rückstellelement 110 besteht. Anstelle eines weiteren Rückstellelements 120 kann aber auch ein Krafteinleitungsmittel 122, beispielsweise ein Betätigungsdraht zur aktiven Betätigung der Orthese 100 hindurchgeführt werden.

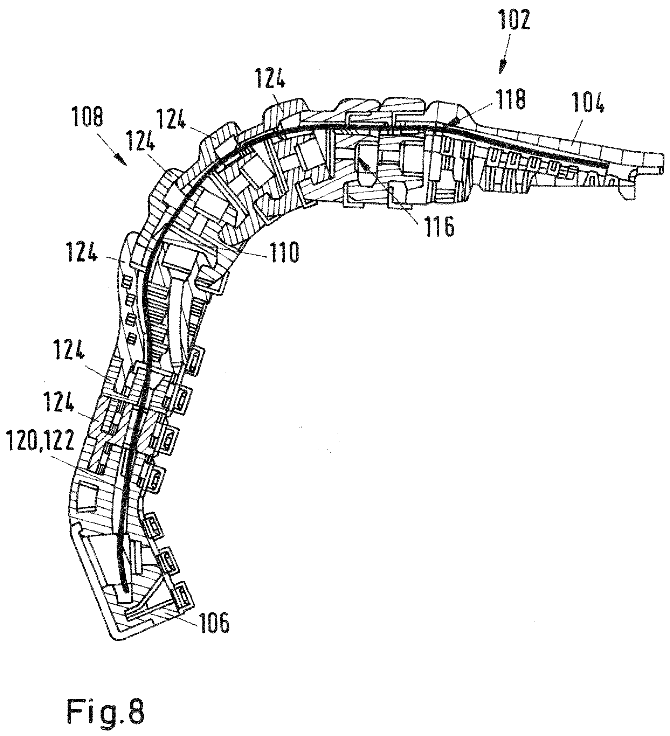

Figur 8 verweist abschließend noch auf die Möglichkeit, dass das Rückstellelement 110 ausschließlich durch die Durchtritte 118 hindurchgeführt sein kann, sodass der Tunnel 116 ungenutzt bleibt. Das in Figur 8 gezeigte Rückstellelement 110 erstreckt sich dabei von dem ersten Orthesenglied 104 (rechts oben) bis zum zweiten Orthesenglied 106 (links unten).

Insgesamt zeichnet sich vorliegende Erfindung dadurch aus, dass verbesserte mechanische Eigenschaften der Orthese 100 bereitgestellt sind, welche sowohl bei aktiv betätigbaren Orthesen, die einen Antrieb aufweisen, als auch bei passiven Orthesen, die antriebslos gestaltet sind, genutzt werden können.

### BEZUGSZEICHENLISTE

- 100: Orthese
- 102: Orthesenteil
- 104: erstes Orthesenglied
- 106: zweites Orthesenglied
- 108: Kopplungsteil
- 110: Rückstellelement
- 112: erste Tasche
- 114: zweite Tasche
- 116: Tunnel
- 118: Durchtritt
- 120: weiteres Rückstellelement
- 122: Krafteinleitungsmittel
- 124: Koppelglied
- 126: Bajonettkulisse
- 128: Bajonetthaken
- 130: Unterarmschiene

## Patentansprüche

1. Orthesenteil (102) für eine Orthese (100), umfassend:
- ein erstes Orthesenglied (104),
- ein zweites Orthesenglied (106),
- ein Kopplungsteil (108), durch welches das erste Orthesenglied (104) mit dem zweiten Orthesenglied (106) begrenzt beweglich verbunden ist, und
- ein Rückstellelement (110),
welches einenends mit dem ersten Orthesenglied (104) und anderenends mit dem zweiten Orthesenglied (106) verbunden ist,
welches aus einer Legierung umfassend Titan und Nickel gebildet ist,
und welches aufgrund seiner elastischen Materialeigenschaften dann eine Rückstellkraft bereitstellt, wenn das erste Orthesenglied (104) relativ bezüglich dem zweiten Orthesenglied (106) ausgelenkt wird,
wobei in dem ersten Orthesenglied (104) eine erste Tasche (112) ausgebildet ist, wobei in dem zweiten Orthesenglied (106) eine zweite Tasche (114) ausgebildet ist, und wobei das Rückstellelement (110) einenends in der ersten Tasche (112) und anderenends in der zweiten Tasche (114) aufgenommen ist,
**dadurch gekennzeichnet, dass** in jeder der Taschen (112, 114) ein Teil eines zweiten Rückstellelements aufgenommen ist, und dass das zweite Rückstellelement aus derselben Legierung wie das Rückstellelement (110) besteht.

2. Orthesenteil (102) nach Anspruch 1, **dadurch gekennzeichnet, dass** die Legierung außerdem Kobalt umfasst.

3. Orthesenteil (102) nach Anspruch 1 oder 2, **dadurch gekennzeichnet, dass** die Legierung des Rückstellelements (110) als Austenit oder überwiegend als Austenit vorliegt.

4. Orthesenteil (102) nach einem der Ansprüche 1 bis 3, **dadurch gekennzeichnet, dass** das Rückstellelement (110) und das zweite Rückstellelement dieselbe Querschnittsform aufweisen.

5. Orthesenteil (102) nach einem der Ansprüche 1 bis 4, **dadurch gekennzeichnet, dass** das mindestens eine Rückstellelement (110) im Wesentlichen röhrenförmig gebildet ist.

6. Orthesenteil (102) nach einem der Ansprüche 1 bis 5, **dadurch gekennzeichnet, dass** in dem Kopplungsteil (108) ein Tunnel (116) für das Rückstellelement (110) ausgebildet ist, und dass das mindestens eine Rückstellelement (110) durch den Tunnel (116) hindurchgeführt ist.

7. Orthesenteil (102) nach einem der Ansprüche 1 bis 6, **dadurch gekennzeichnet, dass** in dem Kopplungsteil (108) ein Durchtritt (118) für ein weiteres Rückstellelement (120) ausgebildet ist, und dass das weitere Rückstellelement (120) durch den Durchtritt (118) hindurchgeführt ist.

8. Orthesenteil (102) nach Anspruch 7, **dadurch gekennzeichnet, dass** das weitere Rückstellelement (120) aus derselben Legierung wie das Rückstellelement (110) besteht.

9. Orthesenteil (102) nach einem der Ansprüche 1 bis 8, **dadurch gekennzeichnet, dass** in dem Kopplungsteil (108) ein Durchtritt (118) für ein Krafteinleitungsmittel (122) zur aktiven Betätigung der Orthese (100) ausgebildet ist, und dass das Krafteinleitungsmittel (122) durch den Durchtritt (118) hindurchgeführt ist.

10. Orthesenteil (102) nach einem der Ansprüche 1 bis 9, **dadurch gekennzeichnet, dass** das Kopplungsteil (108) aus einer Mehrzahl von Koppelgliedern (124) gebildet ist, die untereinander begrenzt beweglich miteinander verbunden sind.

11. Orthesenteil (102) nach Anspruch 10, **dadurch gekennzeichnet, dass** sich ein Tunnel (116) für das Rückstellelement durch alle Koppelglieder (124) des Kopplungsteils (108) hindurch erstreckt.

12. Orthesenteil (102) nach Anspruch 10 oder 11, **dadurch gekennzeichnet, dass** sich ein Durchtritt (118) für ein weiteres Rückstellelement (120) und/oder für ein Krafteinleitungsmittel (122) zur aktiven Betätigung der Orthese (100) durch alle Koppelglieder (124) des Kopplungsteils (108) hindurch erstreckt.

13. Orthesenteil (102) nach einem der Ansprüche 10 bis 12, **dadurch gekennzeichnet, dass** jedes der Koppelglieder (124) eine an einer ersten Stirnseite ausgebildete Bajonettkulisse (126) aufweist,
dass jedes der Koppelglieder (124) einen zu der Bajonettkulisse (126) korrespondierenden Bajonetthaken (128) aufweist, der an einer der ersten Stirnseite gegenüberliegenden zweiten Stirnseite ausgebildet ist,
und dass zwischen je zwei der Koppelglieder (124) oder zwischen einem der Koppelglieder (124) und einem der Orthesenglieder (104, 106) eine begrenzt bewegliche Bajonettverbindung ausgebildet ist.

14. Bausatz zur Herstellung eines Orthesenteils (102) nach einem der Ansprüche 1 bis 13, umfassend mindestens ein erstes Orthesenglied (104), mindestens ein zweites Orthesenglied (106) und mindestens ein Rückstellelement (110), wobei das erste Orthesenglied (104) mit dem zweiten Orthesenglied (106) verbindbar ist, welches aus einer Legierung umfassend Titan und Nickel gebildet ist, und welches aufgrund seiner elastischen Materialeigenschaften dann eine Rückstellkraft bereitstellt, wenn im montierten Zustand des Orthesenteils (102) das erste Orthesenglied (104) relativ bezüglich dem zweiten Orthesenglied (106) ausgelenkt wird, wobei in dem ersten Orthesenglied (104) eine erste Tasche (112) ausgebildet ist, wobei in dem zweiten Orthesenglied (106) eine zweite Tasche (114) ausgebildet ist, und wobei das Rückstellelement (110) einenends in der ersten Tasche (112) und anderenends in der zweiten Tasche (114) aufgenommen werden kann, wobei in jeder der Taschen (112, 114) ein Teil eines zweiten Rückstellelements aufgenommen werden kann und das zweite Rückstellelement aus derselben Legierung wie das Rückstellelement (110) besteht.

15. Orthese (100) mit einem Orthesenteil (102) nach einem der Ansprüche 1 bis 13.

## Claims

1. An orthosis part (102) for an orthosis (100), comprising:
- a first orthosis element (104)
- a second orthosis element (106),
- a coupling part (108), by which the first orthosis element (104) is connected to the second orthosis element (106) in a limitedly movable manner, and
- a resetting element (110),
which is connected at one end to the first orthosis element (104) and at the other end to the second orthosis element (106),
which is formed from an alloy comprising titanium and nickel,
and which, due to its elastic material properties, provides a restoring force when the first orthosis element (104) is deflected relative to the second orthosis element (106),
wherein a first pocket (112) is formed in the first orthosis element (104), wherein a second pocket (114) is formed in the second orthosis element (106), and wherein the resetting element (110) is received at one end in the first pocket (112) and at the other end in the second pocket (114),
**characterized in that** a part of a second resetting element is accommodated in each of the pockets (112, 114), and **in that** the second resetting element consists of the same alloy as the resetting element (110).

2. The orthosis part (102) according to claim 1, **characterized in that** the alloy further comprises cobalt.

3. The orthosis part (102) according to claim 1 or 2, **characterized in that** the alloy of the resetting element (110) is present as austenite or predominantly as austenite.

4. The orthosis part (102) according to any one of claims 1 to 3, **characterized in that** the resetting element (110) and the second resetting element have the same cross-sectional shape.

5. The orthosis part (102) according to any one of claims 1 to 4, **characterized in that** the at least one resetting element (110) is formed substantially tubular.

6. The orthosis part (102) according to any one of claims 1 to 5, **characterized in that** a tunnel (116) for the resetting element (110) is formed in the coupling part (108), and **in that** the at least one resetting element (110) is guided through the tunnel (116).

7. The orthosis part (102) according to any one of claims 1 to 6, **characterized in that** a passage (118) for a further resetting element (120) is formed in the coupling part (108), and **in that** the further resetting element (120) is guided through the passage (118).

8. The orthosis part (102) according to claim 7, **characterized in that** the further resetting element (120) consists of the same alloy as the resetting element (110).

9. The orthosis part (102) according to any one of claims 1 to 8, **characterized in that** a passage (118) for a force introduction means (122) for active actuation of the orthosis (100) is formed in the coupling part (108), and **in that** the force introduction means (122) is guided through the passage (118).

10. The orthosis part (102) according to any one of claims 1 to 9, **characterized in that** the coupling part (108) is formed from a plurality of coupling elements (124), which are connected to each other in a limitedly movable manner.

11. The orthosis part (102) according to claim 10, **characterized in that** a tunnel (116) for the resetting element extends through all coupling elements (124) of the coupling part (108).

12. The orthosis part (102) according to claim 10 or 11, **characterized in that** a passage (118) for a further resetting element (120) and/or for a force introduction means (122) for active actuation of the orthosis (100) extends through all coupling elements (124) of the coupling part (108).

13. The orthosis part (102) according to any one of claims 10 to 12, **characterized in that** each of the coupling elements (124) has a bayonet link (126) formed on a first end face,
**in that** each of the coupling elements (124) has a bayonet hook (128) which corresponds to the bayonet link (126) and is formed on a second end face opposite the first end face,
and **in that** a bayonet connection with limited movement is formed between two of the coupling elements (124) or between one of the coupling elements (124) and one of the orthosis elements (104, 106).

14. A kit for manufacturing an orthosis part (102) according to any one of claims 1 to 13, comprising at least one first orthosis element (104), at least one second orthosis element (106) and at least one resetting element (110), wherein the first orthosis element (104) is connectable to the second orthosis element (106), which is formed from an alloy comprising titanium and nickel, and which, due to its elastic material properties, provides a restoring force when the first orthosis element (104) is deflected relative to the second orthosis element (106) in the mounted state of the orthosis part (102), wherein a first pocket (112) is formed in the first orthosis element (104), wherein a second pocket (114) is formed in the second orthosis element (106), and wherein the resetting element (110) can be received at one end in the first pocket (112) and at the other end in the second pocket (114), wherein a part of a second resetting element can be received in each of the pockets (112, 114) and the second resetting element consists of the same alloy as the resetting element (110).

15. An orthosis (100) comprising an orthosis part (102) according to any one of claims 1 to 13.

## Revendications

1. Pièce d'orthèse (102) pour une orthèse (100), comprenant :
- un premier membre d'orthèse (104),
- un second membre d'orthèse (106),
- une pièce de couplage (108), par l'intermédiaire de laquelle le premier membre d'orthèse (104) est relié avec une mobilité limitée au second membre d'orthèse (106), et
- un élément de rappel (110)
qui est relié à une extrémité au premier membre d'orthèse (104) et à l'autre extrémité au second membre d'orthèse (106),
qui est formé d'un alliage comprenant du titane et du nickel et qui, en raison de ses propriétés de matériau élastiques, fournit alors une force de rappel lorsque le premier membre d'orthèse (104) est dévié par rapport au second membre d'orthèse (106),
dans laquelle une première poche (112) est formée dans le premier membre d'orthèse (104), dans laquelle une seconde poche (114) est formée dans le second membre d'orthèse (106), et dans laquelle l'élément de rappel (110) est logé à une extrémité dans la première poche (112) et à l'autre extrémité dans la seconde poche (114),
**caractérisée en ce qu'**une partie d'un second élément de rappel est logée dans chacune des poches (112, 114), et **en ce que** le second élément de rappel se compose du même alliage que celui de l'élément de rappel (110).

2. Pièce d'orthèse (102) selon la revendication 1, **caractérisée en ce que** l'alliage comprend en outre du cobalt.

3. Pièce d'orthèse (102) selon la revendication 1 ou 2, **caractérisée en ce que** l'alliage de l'élément de rappel (110) se présente en tant qu'austénite ou principalement en tant qu'austénite.

4. Pièce d'orthèse (102) selon l'une quelconque des revendications 1 à 3, **caractérisée en ce que** l'élément de rappel (110) et le second élément de rappel présentent la même forme de section transversale.

5. Pièce d'orthèse (102) selon l'une quelconque des revendications 1 à 4, **caractérisée en ce que** le au moins un élément de rappel (110) est formé sensiblement de forme tubulaire.

6. Pièce d'orthèse (102) selon l'une quelconque des revendications 1 à 5, **caractérisée en ce qu'**un tunnel (116) pour l'élément de rappel (110) est formé dans la pièce de couplage (108), et **en ce que** le au moins un élément de rappel (110) est guidé à travers le tunnel (116).

7. Pièce d'orthèse (102) selon l'une quelconque des revendications 1 à 6, **caractérisée en ce qu'**un passage (118) pour un élément de rappel supplémentaire (120) est formé dans la pièce de couplage (108), et **en ce que** l'élément de rappel supplémentaire (120) est guidé à travers le passage (118).

8. Pièce d'orthèse (102) selon la revendication 7, **caractérisée en ce que** l'élément de rappel supplémentaire (120) se compose du même alliage que celui de l'élément de rappel (110).

9. Pièce d'orthèse (102) selon l'une quelconque des revendications 1 à 8, **caractérisée en ce qu'**un passage (118) pour un moyen d'introduction de force (122) pour l'actionnement actif de l'orthèse (100) est formé dans la pièce de couplage (108), et **en ce que** le moyen d'introduction de force (122) est guidé à travers le passage (118).

10. Pièce d'orthèse (102) selon l'une quelconque des revendications 1 à 9, **caractérisée en ce que** la pièce de couplage (108) est formée d'une pluralité de membres de couplage (124) qui sont reliés entre eux avec une mobilité limitée.

11. Pièce d'orthèse (102) selon la revendication 10, **caractérisée en ce qu'**un tunnel (116) pour l'élément de rappel s'étend à travers tous les membres de couplage (124) de la pièce de couplage (108).

12. Pièce d'orthèse (102) selon la revendication 10 ou 11, **caractérisée en ce qu'**un passage (118) pour un élément de rappel supplémentaire (120) et/ou pour un moyen d'introduction de force (122) pour l'actionnement actif de l'orthèse (100) s'étend à travers tous les membres de couplage (124) de la pièce de couplage (108).

13. Pièce d'orthèse (102) selon l'une quelconque des revendications 10 à 12, **caractérisée en ce que** chacun des membres de couplage (124) présente une coulisse à baïonnette (126) formée sur une première face avant, **en ce que** chacun des membres de couplage (124) présente un crochet à baïonnette (128) correspondant à la coulisse à baïonnette (126) qui est formé sur une seconde face avant opposée à la première face frontale, et **en ce qu'**une connexion à baïonnette à mobilité limitée est formée entre deux des membres de couplage (124) ou entre un des membres de couplage (124) et un des membres d'orthèse (104, 106).

14. Kit pour la fabrication d'une pièce d'orthèse (102) selon l'une quelconque des revendications 1 à 13, comprenant au moins un premier membre d'orthèse (104), au moins un second membre d'orthèse (106) et au moins un élément de rappel (110), dans lequel le premier membre d'orthèse (104) peut être relié au second membre d'orthèse (106) qui est formé d'un alliage comprenant du titane et du nickel et qui, en raison de ses propriétés de matériau élastiques, fournit alors une force de rappel lorsque, à l'état monté de la pièce d'orthèse (102), le premier membre d'orthèse (104) est dévié par rapport au second membre d'orthèse (106), dans lequel une première poche (112) est formée dans le premier membre d'orthèse (104), dans lequel une seconde poche (114) est formée dans le second membre d'orthèse (106), et dans lequel l'élément de rappel (110) peut être logé à une extrémité dans la première poche (112) et à l'autre extrémité dans la seconde poche (114), dans lequel une partie d'un second élément de rappel peut être logé dans chacune des poches (112, 114), et le second élément de rappel se compose du même alliage que celui de l'élément de rappel (110).

15. Orthèse (100) avec une pièce d'orthèse (102) selon l'une quelconque des revendications 1 à 13.
